# EUROPEAN PATENT SPECIFICATION

(11) **EP 3 274 036 B1**
(45) Date of publication and mention of the grant of the patent: **19.02.2020**
(21) Application number: 16714231.4
(22) Date of filing: 22.03.2016
(51) Int. Cl.: A61M 25/00, A61M 19/00, A61M 29/02, A61M 25/06, A61M 25/10

(54) **BALLOON-TIPPED CATHETER FOR CONTINUOUS NERVE BLOCKS**
KATHETER MIT BALLONSPITZE FÜR KONTINUIERLICHE NERVENBLOCKIERUNGEN
CATHÉTER À BALLONNET POUR BLOCS NERVEUX CONTINUS

(30) Priority: 25.03.2015 DK 201570169
(43) Date of publication of application: 31.01.2018
(73) Proprietor: Rigshospitalet, Copenhagen University Hospital, 2100 Copenhagen Ø (DK)
(72) Inventor: HESSELBJERG, Lars, 2100 Copenhangen Ø (DK)
(74) Representative: Plougmann Vingtoft a/s
(86) International application number: PCT/DK2016/050089
(87) International publication number: WO 2016/150448

(56) References cited:
- WO-A1-97/37714
- US-A1- 2002 095 114
- US-A1- 2010 249 749

## Description

### FIELD OF THE INVENTION

The present invention relates to a catheter for administering anaesthetics to a peripheral nerve site in a patient, and in particular to a catheter having an inflatable balloon arranged at the end of the catheter placed at the peripheral nerve site during use.

### BACKGROUND OF THE INVENTION

Administering of anaesthetics to a peripheral nerve site in a patient is relevant in a number of situations including in order to relieve pain following a surgery; such a pain will typically last for days. This is also referred to as a nerve block and results in a deliberate and temporary interruption of signals travelling along a nerve with the purpose of pain relief. If the anaesthetics is to be delivered over a period of time, it can be done by use of a catheter inserted into the patient via a needle which is subsequently removed. The outer end of the catheter is then fastened to the skin, but the patient's movement can easily result in the tip of the catheter moving away from the nerve so that it no longer works as intended. Therefore, the use of nerve catheters is diminished in favour of a single shot technique where one dose of anaesthetics is injected through a needle which is then removed. This procedure is rather simple in terms of e.g. sterility demands, but such a nerve block will typically last for no longer than 4 to 12 hours depending on the type of anaesthetics used. In some cases this is long enough whereas e.g. pain following a surgery often lasts for much longer.

It is known in relation to cardiac monitoring to use a catheter comprising an inflatable balloon; such a catheter is known as a Swan-Ganz catheter. The balloon is transferred by the bloodstream from the heart through the lung vessels. When the balloon reaches a location where its size corresponds to the size of the blood vessels, the catheter is held in place, and it is possible to perform measurements of the pressures in the heart. Such a catheter has two or more injection ports, and it is introduced into the bloodstream by the Seldinger technique using a guide wire and a dilator. For the introduction of a catheter for peripheral nerve block, the use of a guide wire is not possible or very impractical because the catheter is placed in solid tissue and not blood vessels. Furthermore, the dimensions of such catheters are too large to be used in combination with the needles used for peripheral nerve blocks. Thus, this technique is not suitable for the placement of nerve catheters.

When peripheral nerve catheters are placed through a needle, the advancement through the patient and the positioning at the nerve site may be followed by use of ultrasound. The needle and the local anaesthetics injected through it are visible on the ultrasound image, but the catheter and especially the tip thereof is typically not. To make the catheter visible on ultrasound, some recently developed catheters have small bubbles of air in the wall. The tip is to be placed close to the nerve, so great care is to be taken not to damage the nerve and to ensure that the distance is still small enough to make the anaesthetics work as intended. WO98/33547 discloses a formerly used technique used to ensure a correct insertion of a catheter for peripheral nerve site anaesthetics. The method described therein uses a nerve stimulator needle and a nerve stimulator electrode, which electrode is used to verify correct insertion and placement of the catheter before the start of the local anaesthetics infusion. The nerve stimulation technique is almost obsolete and very seldom used. The catheter disclosed may further have an immobilizing structure in the form of an inflatable balloon which is attached at some point along the shaft of the catheter, typically mid-shaft. Inflation thereof results in the catheter becoming snugged against the posterior surface of the pectoralis minor muscle tendon. Hereby the part of the catheter close to the balloon keeps its position, but there is still some risk of the tip of the catheter moving away from the nerve site so that it does not deliver the anaesthetics to the desired nerve site.

US 2011/0071497A1 discloses a balloon fixated catheter for the delivery of medication for the relief of pain. It has the balloon placed at the distal end of the catheter tube, the balloon being inflatable to a size sufficient to retain the catheter within the area of placement. The catheter has one tube for inflation of the balloon and another tube for infusion of medication. These two tubes are independently produced and then joined to provide a unitary catheter.

A further challenge with peripheral nerve blocks exists especially when the nerve to be blocked is close to the skin. In that case, the local anaesthetics being injected will often go backwards through the canal made by the needle instead of being placed around the nerve. It may then end under the dressing used to fixate the catheter to the skin.

Hence, an improved catheter for administering anaesthetics to a peripheral nerve site in a patient would be advantageous, and in particular a more efficient and/or reliable catheter would be advantageous.

### OBJECT OF THE INVENTION

It is an object of the present invention to provide a catheter for administering anaesthetics to a peripheral nerve site in a patient with which the certainty that the tip of the catheter at the peripheral nerve site stays at the desired position can be significantly increased compared to known catheters.

It is another object of the present invention to provide such a catheter which is easier and more secure to arrange precisely at the desired position at the peripheral nerve site compared to known catheters.

It is an object of at least some embodiments of the present invention to provide such a catheter with which the risk of the anaesthetics unintentionally leaking into the tissue surrounding the peripheral nerve site can be decreased compared to known catheters.

It is another object of at least some embodiments of the present invention to provide a catheter with which it is easier to ensure that the medication can be delivered very close to the nerve to be blocked.

It is another object of at least some embodiments of the present invention to provide a catheter with which the risk of unintentionally blocking the delivery of the anaesthetics by the balloon is lowered.

It is a further object of the present invention to provide an alternative to the prior art.

In particular, it may be seen as an object of the present invention to provide a catheter for administering anaesthetics to a peripheral nerve site in a patient that solves the above mentioned problems of the prior art.

### SUMMARY OF THE INVENTION

The above-described objects and several other objects are intended to be obtained in a first aspect of the invention by providing a catheter for administering anaesthetics to a peripheral nerve site in a patient, the catheter comprising:
- a hollow, flexible catheter tube having a first inner lumen extending along a longitudinal axis of the catheter tube from a proximal end to a distal end, the distal end being arranged at the peripheral nerve site when the catheter is in use, and
- a balloon adapted to be inflated from a collapsed state to an inflated state by a fluid fed through the catheter tube into an inner cavity of the balloon, wherein the balloon is arranged at the distal end of the catheter tube, and the inner cavity of the balloon is in fluid communication with the proximal end of the catheter tube via the inner lumen.

By "lumen" is preferably meant an open area inside an object. In the present context, the lumen is the hollow cavity surrounded by the wall of the catheter tube.

"Fluid" may be a gas, such as air, or a liquid, such as salt water or the anaesthetics.

The specifying that the distal end of the catheter is to be arranged *at* the peripheral nerve site is mainly made to define which end of the catheter that is called the distal end. By "the peripheral nerve site" is preferably meant the region surrounding the nerve that is to be blocked without stating a specific distance from the nerve itself. It will be well-known to a person skilled in the art how close to the nerve the anaesthetics is to be delivered to work as intended.

By specifying that the balloon is arranged *at* the distal end is preferably meant proximate to the distal end, such as having a surface close to but not necessarily touching the end surface of the catheter. There may e.g. be a few mm of distance from the end of the catheter and to the closest outer surface of the balloon. Since the balloon has a certain extension, such as extending over 5 to 20 mm of the length of the catheter, it is clear that "at the distal end" is not intended to refer to a point. The balloon may also be an elongate balloon extending over more than 20 mm along the length of the catheter, such as extending from the end and 25 to 40 mm along the length.

In a second aspect the invention relates to a needle-catheter kit for administering anaesthetics to a peripheral nerve site in a patient, the kit comprising:
- a catheter according to the invention and having an outer diameter which allows for advancement through the needle with the balloon in the collapsed state, and
- a needle adapted to be partly inserted into a patient and positioned so that it extends from the outside of the patient and to the peripheral nerve site where the anaesthetics is to be administered, the needle having a hollow shaft through which the catheter can be advanced.

In an alternative embodiment, the second aspect of the invention relates to a needle-catheter kit for administering anaesthetics to a peripheral nerve site in a patient, the kit comprising:
- a catheter according to the invention and having an inner diameter of the catheter tube which allows for insertion of the needle in the catheter tube and for removal of the needle through the catheter tube after positioning the catheter in the patient, and
- a needle adapted to be partly inserted into a patient and positioned so that it extends from the outside of the patient and to the peripheral nerve site where the anaesthetics is to be administered, the needle having a hollow shaft over which the catheter can be arranged.

In yet another alternative embodiment, the second aspect of the invention relates to a needle-catheter kit for administering anaesthetics to a peripheral nerve site in a patient, the kit comprising:
- an introducer comprising a hollow tubular introducer tube, the introducer tube having an inner diameter which allows for insertion of the needle into the introducer tube and for removal of the needle through the introducer tube after positioning at least part of the introducer tube and part of the needle in the patient,
- a catheter as described above and having an outer diameter which allows for advancement through the introducer tube with the balloon in the collapsed state, and
- a needle adapted to be inserted into the introducer tube and partly inserted into a patient and positioned so that it extends from the outside of the patient and to the peripheral nerve site where the anaesthetics is to be administered.

Reference(s) to "embodiment(s)" throughout the description which are not under the scope of the appended claims merely represent possible exemplary executions and are therefore not part of the present invention.

A third aspect relates to a method of administering anaesthetics to a peripheral nerve site in a patient; the method, which is not part of the invention comprising the following steps:
- inserting a needle into the patient and positioning it so that it extends from the outside of the patient and to the peripheral nerve site where the anaesthetics is to be administered,
- advancing the distal end of a catheter as described above and in the following through the needle to the peripheral nerve site,
- inflating the balloon to maintain the distal end of the catheter at a desired position,
- removing the needle, and
- administering anaesthetics to the peripheral nerve site.

In an alternative embodiment, the third aspect relates to a method of administering anaesthetics to a peripheral nerve site in a patient, the method comprising the following steps:
- inserting a catheter as described above and in the following into a needle,
- inserting the needle with the catheter into the patient and positioning it so that it extends from the outside of the patient and to the peripheral nerve site where the anaesthetics is to be administered,
- inflating the balloon to maintain the distal end of the catheter at a desired position,
- removing the needle, and
- administering anaesthetics to the peripheral nerve site.

In another alternative embodiment, the third aspect relates to a method of administering anaesthetics to a peripheral nerve site in a patient, the method comprising the following steps:
- inserting a needle into the catheter tube of a catheter as described above and in the following,
- inserting the needle with the catheter into the patient and positioning it so that it extends from the outside of the patient and to the peripheral nerve site where the anaesthetics is to be administered,
- inflating the balloon to maintain the distal end of the catheter at a desired position,
- removing the needle through the catheter tube, and
- administering anaesthetics to the peripheral nerve site.

Such a method where the needle is inserted into the catheter tube is typically referred to as an "over the needle" technique. The step of inserting the needle into the catheter tube may take place as part of the manufacturing, or it may be performed by the person performing the nerve block or an assistant preparing the nerve block. To enable the inflation of the balloon, it is typically necessary to put a connector onto the proximal end of the catheter tube through which connector the catheter tube is connected to the supply of fluid used for the inflation. In order to be able to remove the needle, it is in some embodiments of the invention necessary to remove the connector again which may cause at least partial deflation of the balloon. After removal of the needle, the catheter tube is connected to the supply of anaesthetics, typically via a connector. By inflating the balloon before the needle is removed, it can be carefully checked that the tip of the catheter is arranged at the desired position at the peripheral nerve site, before the administering of the anaesthetics is initiated.

The balloon may be kept inflated during part of the removal of the needle, e.g. until the end of the needle reaches the connector, to help maintaining the catheter in place during retraction of the needle.

In embodiments where the needle is inserted into the patient before the catheter is advanced through needle, a small amount of anaesthetics is typically injected through the needle before the catheter is advanced through the needle. Since the needle is visible on ultrasound, the tip of the needle is typically placed at the desired positon of the catheter during infusion, before the advancement of the catheter is initiated. This means that the needle covers the catheter when in place so that the needle has to be slightly retracted to expose the balloon to enable it to be inflated. Then the correct position is checked again before removal of the needle.

In yet another alternative embodiment, the third aspect relates to a method of administering anaesthetics to a peripheral nerve site in a patient, the method comprising the following steps:
- providing a needle inserted into an introducer tube of an introducer,
- inserting the needle together with the introducer tube into the patient and positioning it so that it extends from the outside of the patient and to the peripheral nerve site where the anaesthetics is to be administered,
- removing the needle while keeping the introducer tube inside the patient,
- advancing the distal end of a catheter as described above through the introducer tube to the peripheral nerve site,
- inflating the balloon to maintain the distal end of the catheter at a desired position,
- retracting the introducer to the outside of the patient, and
- administering anaesthetics to the peripheral nerve site.

Such a method may further comprise fastening the introducer to a connector of the catheter after the retraction, the connector being a part where two lumen of the catheter divide.

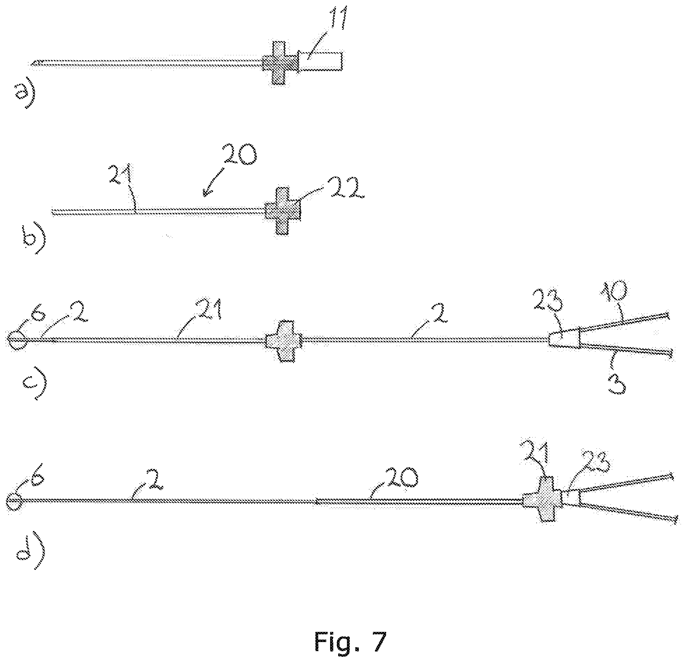

The details and advantages of embodiments of the invention comprising an introducer are described in relation to figure 7.

The actual anaesthetics used in relation to the present invention will be well known to a person skilled in the art.

In relation to surgery, a primary injection of anaesthetics will typically be injected via the needle without the catheter inserted to provide the peripheral nerve block necessary for the surgery. In this respect, it may be relevant to move the needle tip to inject anaesthetics at more points around the location of the surgery to be performed. The needle tip is then arranged at the correct position for the placement of the catheter, and the distal end of the catheter is advanced through the needle as described above to prepare for the post-surgery pain relief. With such a method it may be obtained that the first injected local anaesthetics provides a volume of liquid inside the tissue which may ease the inflation of the balloon.

An advantage of having the balloon arranged at the distal end of the catheter is that the inflation thereof results in the distal end being kept at the desired position inside the patient so that the administering of the anaesthetics is maintained at the peripheral nerve site even if the patient moves. Known catheters are typically fastened to the skin of the patient at the proximal end which means that it may still move at the distal end. The possibility of ensuring that the tip of the catheter stays in place enables continuous infusion of the local anaesthetics close to the nerve so that the patient can be kept free from pain for as long as needed, even for days.

A further advantage of the balloon is that it can provide a plugging effect by preventing that the anaesthetics runs into the tissue surrounding the point of administration, such as an end hole of a known catheter.

The way the needle is inserted in the patient, the way the primary block for surgery is done, and the subsequent advancement of the catheter inside the needle is considered to be known to a person skilled in the art.

The fluid used for inflating the balloon from the collapsed to the inflated state may typically be air or salt water, but it may also be the anaesthetics itself. These possibilities will be described in more details in the following and in relation to the drawings.

In presently preferred embodiments of the invention, the first inner lumen may be the only lumen extending along the longitudinal axis of the catheter tube. In such an embodiment, this lumen is used both for the fluid used to inflate the balloon and for the subsequent administering of anaesthetics. Such a catheter tube may be easier to manufacture, especially for small diameters, than one having two lumen as will be described below. First the balloon is inflated carefully, and then it is connected to an infuser system for the administering of the anaesthetics.

The catheter tube may further comprise perforations in a wall along a section near the distal end and surrounded by the balloon so that the anaesthetics can flow from the catheter tube and into the balloon through the perforations. Hereby it is possible to use injected anaesthetics to inflate the balloon so that no further types of fluid needs to be injected into the patient.

In embodiments comprising such perforations in the wall, the catheter tube may further comprise at least one end hole at the distal end, the end hole being significantly smaller than the perforations in the wall and not surrounded by the balloon so that injection of the anaesthetics results in inflation of the balloon until a certain pressure therein is reached where after the subsequently administered anaesthetics leaves the inner lumen through the end hole. A catheter with only one lumen and functioning as described above has the advantages of being simple to design and produce because of the catheter on its own and because only one connector will be needed. This is an advantage over catheters with two lumen made from individual tubes that are subsequently joined, thus adding an extra manufacturing step. Furthermore, the appearance will resemble those of a traditionally used catheter which may be of importance to the users.

In an alternative embodiment of the invention, the balloon may be made from a material having permeability properties enabling that liquid anaesthetics can permeate the balloon when the pressure inside the balloon exceeds a certain value. Such permeability properties may be obtained via the materials from which the balloon is made, or they may be obtained via very small perforations in the wall of the balloon. When this is used in combination with the catheter tube comprising perforations in a wall along a section near the distal end and surrounded by the balloon so that the liquid anaesthetics can flow from the catheter tube and into the balloon through the perforations, it can be obtained that the injected anaesthetics first inflates the balloon until a certain pressure is obtained in the balloon. Hereby the balloon is fixated at the desired position in the patient. Then the anaesthetics starts penetrating the balloon to spread into the tissue at the peripheral nerve site. Hereby it is obtained that the anaesthetics is delivered over a larger area than in the embodiment where it only leaves the catheter through an end hole. If the infusion rate of the anaesthetics is increased, the size of the balloon may increase slightly until a new equilibrium state is reached.

In both of the two last-mentioned embodiments it is obtained that the balloon can provide a blocking of the canal made by the needle during insertion, so that anaesthetics is prevented from running into the canal and away from the peripheral nerve site where it is intended to work.

A catheter tube as described above may further comprise a second inner lumen arranged substantially parallel to the first inner lumen and extending from the proximal end to the distal end of the catheter tube. In such embodiments comprising two lumen, they may be arranged next to each other or so that the one surrounds the other. One of the lumen can then be used to inflate the balloon, and one can be used for the anaesthetics. When the balloon has been inflated e.g. by use of air, the lumen used for the inflation can be plugged-off so that the anaesthetics can be injected under very low pressure through the other lumen without risking that the balloon collapses unintentionally as might have happened with a catheter with only one lumen. Such an unintended collapse of the balloon would result in a risk of movement of the distal end as is a problem with some known catheters without a balloon as described in relation to the background of the invention. The method used to plug-off the lumen and the balloon will be well-known to a person skilled in the art.

In the embodiments having a second lumen, the catheter tube may be impermeable to the anaesthetics along the whole length so that the anaesthetics is to leave the catheter tube through an end hole at the distal end, the end hole not being surrounded by the balloon.

In preferred embodiments as described above, the balloon may be arranged closer to the proximal end of the catheter tube than a point where the anaesthetics is to leave the catheter tube when the catheter is in use. In such a preferred embodiment, the point of delivery of the anaesthetics is at the outer surface of the balloon facing away from the proximal end of the catheter tube. Hereby the risk of the balloon unintentionally blocking the delivery of the anaesthetics is lowered. Such unintentional blocking with catheters having the hole through which the anaesthetics is delivered located before the balloon could e.g. happen if a pulling force was applied to the proximal end of the catheter. Such pulling could e.g. be due to the person having the catheter inserted moving around or the catheter being caught by something.

In presently preferred embodiments of the invention, an outer diameter of the catheter tube may be at most 1 mm. This will resemble the typical size of a known catheter used for peripheral nerve blocks. Hereby it will be possible to advance a catheter according to the present invention inside a known needle typically having an inner diameter of around 1.2 mm, such as 1.219 mm. However, other dimensions are also covered by the present invention. Such dimensions may be an outer diameter of 1 to 5 mm, such as 1.5 to 3 mm or 2 to 4 mm.

The first, second and third aspects, of the present invention may each be combined with any of the other aspects. These and other aspects of the invention will be apparent from and elucidated with reference to the embodiments described hereinafter.

### BRIEF DESCRIPTION OF THE FIGURES

The catheter according to the invention as well as the intended use thereof will now be described in more detail with regard to the accompanying figures. The figures show one way of implementing the present invention and is not to be construed as being limiting to other possible embodiments falling within the scope of the attached claim set.
Figure 1 shows schematically the overall idea of having an inflatable balloon arranged at a distal end of a catheter for administering anaesthetics to a peripheral nerve site in a patient.
Figure 2 shows schematically an embodiment of the invention wherein the catheter comprises perforations in a wall along a section near the distal end and surrounded by the balloon.
Figure 3 shows schematically an embodiment of the invention wherein the catheter tube comprises an end hole at the distal end through which the anaesthetics can flow after the balloon has been inflated.
Figure 4 shows schematically an embodiment of the invention wherein the catheter tube comprises two lumen.
Figure 5 shows schematically a needle for use in a needle-catheter kit according to an aspect of the present invention.
Figure 6 is flow charts showing three methods according to the present invention.
Figure 7 shows schematically the use of a needle-catheter kit comprising an introducer according to some embodiments of the invention.

### DETAILED DESCRIPTION OF AN EMBODIMENT

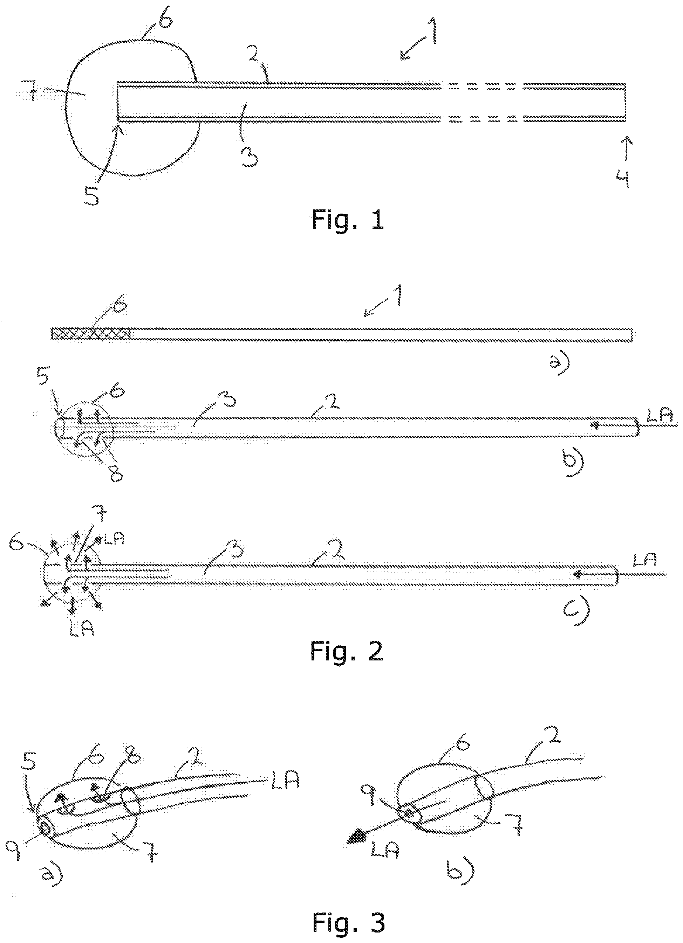

Figure 1 shows schematically the overall idea of having an inflatable balloon arranged at a distal end of a catheter for administering anaesthetics to a peripheral nerve site in a patient. The balloon is in this figure shown as being arranged so that it surrounds the distal end of the catheter. It may also be arranged so that the catheter tube extends beyond the balloon. It may e.g. end at the outer surface of the balloon, or the balloon may be arranged so that there is a small distance to the end of the catheter. With an embodiment where the balloon is located closer to the proximal end of the catheter tube than the hole through which the anaesthetics leaves the inner lumen, the risk of the balloon unintentionally blocking the hole is lowered. Such unintentional blocking with catheters having the hole where the anaesthetics is delivered located before the balloon could e.g. take place if a pulling force was applied to the proximal end of the catheter. The balloon is shown as having a round shape, but it may also have an elongate shape. The catheter 1 comprises a hollow, flexible catheter tube 2 having a first inner lumen 3 extending along a longitudinal axis of the catheter tube 2 from a proximal end 4 to a distal end 5. The distal end 5 is the end which will be arranged at the peripheral nerve site when the catheter 1 is in use. The catheter 1 further comprises a balloon 6 arranged at the distal end 5 of the catheter tube 2. The inner cavity 7 of the balloon 6 is in fluid communication with the proximal end 4 of the catheter tube 2 via the inner lumen 3. The balloon 6 is adapted to be inflated from a collapsed state to an inflated state by a fluid fed through the catheter tube 2 into the inner cavity 7 of the balloon 6; figure 1 shows the inflated state. In the embodiment shown in figure 1, the first inner lumen 3 is the only lumen extending along the longitudinal axis of the catheter tube 2.

The catheter tube 2 can be made from a number of flexible materials, such as silicone, latex, polyamide, Teflon® or polyurethane. It may also be made from two materials, such as having an inner layer of polyamide and an outer layer of polyurethane. The outer surface of the catheter tube 2 should preferably be smooth enough to ease the removal of the needle used for the insertion of the catheter 1. The balloon may e.g. be made from a rubber material, such as silicone or latex.

Figure 2.a shows a catheter 1 with the balloon 6 in the collapsed state. In the embodiment in figure 2, the catheter tube 2 comprises perforations in a wall along a section near the distal end 5 and surrounded by the balloon 6. As shown in figure 2.b, the liquid anaesthetics LA can flow from the catheter tube 2 and into the balloon 6 through the perforations 8 in the wall resulting in an inflation of the balloon 6 due to the higher pressure in the liquid anaesthetics than in the surrounding tissue at the peripheral nerve site. As shown in figure 2.c, the balloon 6 is made from a material having permeability properties enabling that liquid anaesthetics LA can permeate the balloon 6 when the pressure inside the balloon 6 exceeds a certain value. These permeability properties can both be the related to material from which the balloon 6 is made, or they can be because of very small perforations (not shown) in the wall of the balloon 6.

Figure 3 shows schematically an alternative embodiment resembling the one described in relation to figure 2, but without the balloon 6 being permeable. Thus, figure 3.a shows the liquid anaesthetics LA flowing into the balloon 6 via perforations 8 in the wall of the catheter wall. In the embodiment in figure 3, the catheter tube 2 further comprises at least one end hole 9 at the distal end 5. The end hole 9 is significantly smaller than the perforations 8 in the wall and it is not surrounded by the balloon 6. Hereby it is obtained that injection of the anaesthetics results in inflation of the balloon 6 until a certain pressure therein is reached, as shown in figure 3.a. Hereafter the subsequently administered anaesthetics leaves the inner lumen 3 through the end hole 9 as shown in figure 3.b.

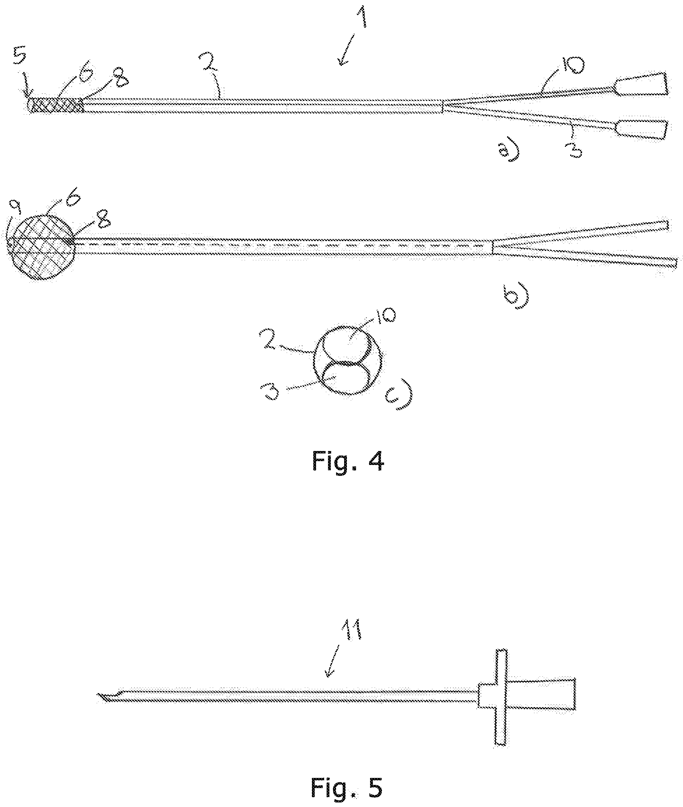

Figure 4 shows schematically an embodiment of the invention wherein the catheter tube 2 comprises two lumen. The second inner lumen 10 is arranged substantially parallel to the first inner lumen 3 and extends from the proximal end 4 to the distal end 5 of the catheter tube 2. The two lumen 3,10 could also be arranged so that the one surrounds the other. Figure 4.a shows the catheter 1 with the balloon 6 in the collapsed state, and figure 4.b shows a close-up of the area around the balloon 6 in the inflated state. Figure 4.c shows schematically the cross-section of the catheter tube 2. One of the lumen 10 is used for the inflation of the balloon 6, and the other lumen 3 is used for the administration of the anaesthetics. In the embodiment shown, the upper lumen 10 (with respect to the orientation of the figure) is for inflation of the balloon 6 via one or more holes 8 in the wall of the catheter tube 2. The other lumen 3 may be arranged so that the catheter tube 2 is impermeable to the anaesthetics along the whole length, so that the anaesthetics is to leave the catheter tube 2 through an end hole 9 at the distal end 5, the end hole 9 not being surrounded by the balloon 6.

Figure 5 shows schematically a needle for use in a needle-catheter kit according to an aspect of the present invention. The kit comprises a catheter 1 as described above and a needle 11 to be used for the arrangement of the catheter in the patient. The catheter 1 may be dimensioned to be advanced inside the hollow shaft of the needle 11. However, in other embodiments, the needle 11 may be arranged inside the catheter 1 during insertion thereof. Apart from this difference, the features of such two embodiments are the same. The needle 11 for such a kit may be identical for one used for traditional nerve blocks.

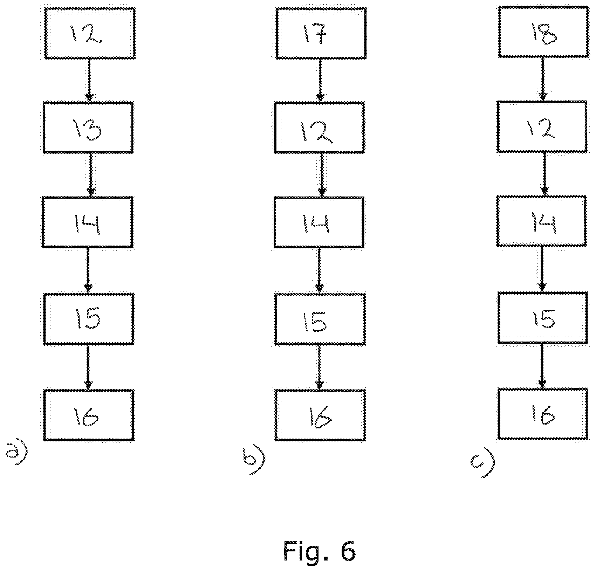

Figure 6 is flow charts showing three method of administering anaesthetics to a peripheral nerve site in a patient according to the present invention. Further details and advantages of the method are given in the section "Summary of the invention" above. In the embodiment shown in figure 6.a, the method comprises the following steps:
- inserting 12 a needle 11 into a patient and positioning it so that it extends from the outside of the patient and to the peripheral nerve site where the anaesthetics is to be administered,
- advancing 13 the distal end 5 of a catheter 1 as described above through the needle 11 to the peripheral nerve site,
- inflating 14 the balloon 6 to maintain the distal end 5 of the catheter 1 at a desired position,
- removing 15 the needle 11, and
- administering 16 anaesthetics to the peripheral nerve site.

In the embodiment shown in figure 6.b, the method comprises the following steps:
- inserting 17 a catheter 1 as described above into a needle 11,
- inserting 12 the needle into a patient and positioning it so that it extends from the outside of the patient and to the peripheral nerve site where the anaesthetics is to be administered with the catheter 1 inserted therein,
- inflating 14 the balloon 6 to maintain the distal end 5 of the catheter 1 at a desired position,
- removing 15 the needle 11, and
- administering 16 anaesthetics to the peripheral nerve site.

In the embodiment shown in figure 6.c, the method comprises the following steps:
- inserting 18 a needle 11 into the catheter tube 2 of a catheter 1 as described above,
- inserting 12 the needle 11 with the catheter 1 into a patient and positioning it so that it extends from the outside of the patient and to the peripheral nerve site where the anaesthetics is to be administered,
- inflating 14 the balloon 6 to maintain the distal end 5 of the catheter 1 at a desired position,
- removing 15 the needle 11 through the catheter tube 2, and
- administering 16 anaesthetics to the peripheral nerve site.

Figure 7 shows schematically the use of a needle-catheter kit comprising an introducer 20 according to some embodiments of the invention. The catheter shown in figure 7 corresponds to the embodiment in figure 4, but an introducer 20 can also be used for other embodiments of the catheter. The introducer 20 comprises a hollow tubular introducer tube 21 which has an inner diameter that allows for insertion of the needle 11 into the introducer tube 21 as shown in figure 7.a. Such a needle 11 is shown in figure 5. The combination of the needle and the introducer may thus also be referred to as an introducing device for the arrangement of the catheter 1 in the patient. The needle 11 is placed in the introducer tube 21 as shown in figure 7.a, and they are inserted into the patient and positioned so that they extends from the outside of the patient to the peripheral nerve site where the anaesthetics is to be administrated in the same way as described above. A small amount of anaesthetics can be administered to the peripheral nerve site through the hollow needle in the introducer 20 to make room for the balloon-tipped catheter to be inserted. The needle 11 is removed again via the introducer tube 21 leaving only the introducer 20 in position in the patient. Then the distal end 5 of the catheter 1 is advanced through the introducer 20 to the peripheral nerve site where the small amount of anaesthetics has been administered. The balloon 6 is inflated via the second inner lumen 10 as described above to maintain the distal end 5 of the catheter 1 at the desired position; this is shown in figure 7.c. After the balloon 6 has been inflated, the introducer 20 is retracted, i.e. passed backwards along the catheter 6, and fastened at the connector 23 of the catheter 1 where the two lumen 3,10 divide. The manual movement of the introducer 20 is made easy by the introducer handle 22 which is also used for the fastening to the connector, e.g. by interference fit or a snap-lock mechanism (not shown). Then the anaesthetics is administered through the first inner lumen 3 in the catheter 1 to the peripheral nerve site. A characteristic of the embodiment in figure 7 is that the introducer 20 does not need to be removed but can be left at the position outside the patient as shown in figure 7.d. The introducer tube 21 is made from a material which is stiff enough for the advancement of the catheter 1 there through and still flexible enough to not cause any disadvantage or risk to the patient as would have been the case if the sharp and stiff needle had not been removed. An advantage of this embodiment comprising an introducer is that it is not necessary to de-couple the connections at the connector 23 from the catheter 1. This would be necessary in order to remove the needle 11 with other embodiments, because the needle cannot pass the connector 23.

Any of the methods described above may typically further comprise a step of injecting an amount of local anaesthetics through the needle to provide a primary block for the surgery. This step may for some embodiments of the invention be performed before advancing of the catheter through the needle.

The investigations made in relation to the present invention included the following test of an embodiment comprising an introducer as described in relation to figure 7. A two-lumen balloon-tipped catheter was inserted in the thigh of the test person. It was inserted into the Adductor canal, where the Saphenus nerve can be blocked. It was inserted under ultrasound guidance and filmed both from the outside and the inside; the inside part by use of ultrasound video. The purposes of the test were not to block the nerve but to show that the balloon at the distal end of the catheter was seen clearly on the ultrasound image and that it would stay in place after hours of walking around. These two things were accomplished and documented on film. Furhtermore, it was also tested that the catheter was not displaced even when having some pulling force applied from the outside. It was showed that this pulling caused slight movement of the balloon and the surrounding tissue, but when the force was released, the balloon was still at the intended position in relation to the peripheral nerve site. Hereby the test showed that the idea of using a catheter for administering anaesthetics to a peripheral nerve site comprising the use of an inflatable balloon provides the desired improvement over prior art.

## Claims

1. Catheter (1) for administering anaesthetics to a peripheral nerve site in a patient, the catheter (1) comprising:
- a hollow, flexible catheter tube (2) having a first inner lumen (3) extending along a longitudinal axis of the catheter tube (2) from a proximal end (4) to a distal end (5), the distal end (5) being arranged at the peripheral nerve site when the catheter (1) is in use, and
- a balloon (6) adapted to be inflated from a collapsed state to an inflated state by a fluid fed through the catheter tube (2) into an inner cavity (7) of the balloon (6),
wherein the balloon (6) is arranged at the distal end (5) of the catheter tube (2), and the inner cavity (7) of the balloon (6) is in fluid communication with the proximal end (4) of the catheter tube (2) via the inner lumen (3),
wherein the catheter tube (2) further comprises a second inner lumen (10) arranged substantially parallel to the first inner lumen (3) and extending from the proximal end (4) to the distal end (5) of the catheter tube (2), and
wherein the balloon (6) is arranged closer to the proximal end (4) of the catheter tube (2) than a point where the anaesthetics is to leave the catheter tube (2) when the catheter is in use.

2. Catheter (1) for administering anaesthetics to a peripheral nerve site in a patient, the catheter (1) comprising:
- a hollow, flexible catheter tube (2) having a first inner lumen (3) extending along a longitudinal axis of the catheter tube (2) from a proximal end (4) to a distal end (5), the distal end (5) being arranged at the peripheral nerve site when the catheter (1) is in use, and
- a balloon (6) adapted to be inflated from a collapsed state to an inflated state by a fluid fed through the catheter tube (2) into an inner cavity (7) of the balloon (6),
wherein the balloon (6) is arranged at the distal end (5) of the catheter tube (2), and the inner cavity (7) of the balloon (6) is in fluid communication with the proximal end (4) of the catheter tube (2) via the inner lumen (3),
wherein the catheter tube (2) further comprises perforations (8) in a wall along a section near the distal end (5) and surrounded by the balloon (6) so that the anaesthetics can flow from the catheter tube (2) and into the balloon (6) through the perforations (9), and
wherein the catheter tube (2) further comprises at least one end hole (9) at the distal end (5), the end hole (9) being significantly smaller than the perforations (8) in the wall and not surrounded by the balloon (6) so that injection of the anaesthetics results in inflation of the balloon (6) until a certain pressure therein is reached where after subsequently administered anaesthetics leaves the inner lumen (3) through the end hole (9).

3. Catheter (1) according to claim 1 or 2, wherein the first inner lumen (3) is the only lumen extending along the longitudinal axis of the catheter tube (2).

4. Catheter (1) according to claim 1, wherein the catheter tube (2) is impermeable to the anaesthetics along the whole length so that the anaesthetics is to leave the catheter tube (2) through an end hole (9) at the distal end (5), the end hole (9) not being surrounded by the balloon (6).

5. Catheter (1) according to any of the preceding claims, wherein an outer diameter of the catheter tube (2) is at most 1 mm.

6. A needle-catheter kit for administering anaesthetics to a peripheral nerve site in a patient, the kit comprising:
- a catheter (1) according to any of the preceding claims and having an outer diameter which allows for advancement through the needle (11) with the balloon (6) in the collapsed state, and
- a needle (11) adapted to be partly inserted into a patient and positioned so that it extends from the outside of the patient and to the peripheral nerve site where the anaesthetics is to be administered, the needle (11) having a hollow shaft through which the catheter (1) can be advanced.

7. A needle-catheter kit for administering anaesthetics to a peripheral nerve site in a patient, the kit comprising:
- a catheter (1) according to any of claims 1-5 and having an inner diameter of the catheter tube (2) which allows for insertion of the needle (11) in the catheter tube (2) and for removal of the needle (11) through the catheter tube (2) after positioning the catheter (1) in the patient, and
- a needle (11) adapted to be partly inserted into a patient and positioned so that it extends from the outside of the patient and to the peripheral nerve site where the anaesthetics is to be administered, the needle (11) having a hollow shaft over which the catheter (1) can be arranged.

8. A needle-catheter kit for administering anaesthetics to a peripheral nerve site in a patient, the kit comprising:
- an introducer (20) comprising a hollow tubular introducer tube (21), the introducer tube (21) having an inner diameter which allows for insertion of the needle (11) into the introducer tube (21) and for removal of the needle (11) through the introducer tube (21) after positioning at least part of the introducer tube (21) and part of the needle (11) in the patient,
- a catheter (1) according to any of claims 1-5 and having an outer diameter which allows for advancement through the introducer tube (21) with the balloon (6) in the collapsed state, and
- a needle (11) adapted to be inserted into the introducer tube (21) and partly inserted into the patient and positioned so that it extends from the outside of the patient and to the peripheral nerve site where the anaesthetics is to be administered.

## Patentansprüche

1. Katheter (1) zum Verabreichen von Anästhetika an eine periphere Nervenstelle bei einem Patienten, wobei der Katheter (1) Folgendes umfasst:
- einen hohlen, flexiblen Katheterschlauch (2) mit einem ersten inneren Lumen (3), das entlang einer Längsachse des Katheterschlauchs (2) von einem proximalen Ende (4) zu einem distalen Ende (5) verläuft, wobei das distale Ende (5) an der peripheren Nervenstelle angeordnet ist, wenn der Katheter (1) in Verwendung ist, und
- einen Ballon (6), der dazu angepasst ist, durch ein Fluid, das durch den Katheterschlauch (2) einem inneren Hohlraum (7) des Ballons (6) zugeführt wird, aus einem zusammengefalteten Zustand in einen aufgeblasenen Zustand aufgeblasen zu werden,
wobei der Ballon (6) an dem distalen Ende (5) des Katheterschlauchs (2) angeordnet ist und der innere Hohlraum (7) des Ballons (6) mit dem proximalen Ende (4) des Katheterschlauchs (2) über das innere Lumen (3) in Fluidkommunikation steht,
wobei der Katheterschlauch (2) ferner ein zweites inneres Lumen (10) umfasst, das im Wesentlichen parallel zu dem ersten inneren Lumen (3) angeordnet ist und von dem proximalen Ende (4) zu dem distalen Ende (5) des Katheterschlauchs (2) verläuft, und
wobei der Ballon (6) näher an dem proximalen Ende (4) des Katheterschlauchs (2) als an einem Punkt, an dem die Anästhetika den Katheterschlauch (2) in Verwendung verlassen sollen, angeordnet ist.

2. Katheter (1) zum Verabreichen von Anästhetika an eine periphere Nervenstelle bei einem Patienten, wobei der Katheter (1) Folgendes umfasst:
- einen hohlen, flexiblen Katheterschlauch (2) mit einem ersten inneren Lumen (3), das entlang einer Längsachse des Katheterschlauchs (2) von einem proximalen Ende (4) zu einem distalen Ende (5) verläuft, wobei das distale Ende (5) an der peripheren Nervenstelle angeordnet ist, wenn der Katheter (1) in Verwendung ist, und
- einen Ballon (6), der dazu angepasst ist, durch ein Fluid, das durch den Katheterschlauch (2) einem inneren Hohlraum (7) des Ballons (6) zugeführt wird, aus einem zusammengefalteten Zustand in einen aufgeblasenen Zustand aufgeblasen zu werden,
wobei der Ballon (6) an dem distalen Ende (5) des Katheterschlauchs (2) angeordnet ist und der innere Hohlraum (7) des Ballons (6) mit dem proximalen Ende (4) des Katheterschlauchs (2) über das innere Lumen (3) in Fluidkommunikation steht,
wobei der Katheterschlauch (2) ferner Perforationen (8) in einer Wand entlang eines Abschnitts nahe dem distalen Ende (5) und durch den Ballon (6) umgeben umfasst, sodass die Anästhetika durch die Perforationen (9) aus dem Katheterschlauch (2) und in den Ballon (6) fließen können, und
wobei der Katheterschlauch (2) ferner zumindest ein Endloch (9) an dem distalen Ende (5) umfasst, wobei das Endloch (9) wesentlich kleiner als die Perforationen (8) in der Wand ist und nicht durch den Ballon (6) umgeben ist, sodass die Injektion der Anästhetika in einem Aufblasen des Ballons (6) resultiert, bis ein bestimmter Druck darin erreicht ist, bei dem danach verabreichte Anästhetika das innere Lumen (3) durch das Endloch (9) verlassen.

3. Katheter (1) nach Anspruch 1 oder 2, wobei das erste innere Lumen (3) das einzige Lumen ist, das entlang der Längsachse des Katheterschlauchs (2) verläuft.

4. Katheter (1) nach Anspruch 1, wobei der Katheterschlauch (2) für die Anästhetika entlang der gesamten Länge undurchlässig ist, sodass die Anästhetika den Katheterschlauch (2) durch ein Endloch (9) an dem distalen Ende (5) verlassen sollen, wobei das Endloch (9) nicht durch den Ballon (6) umgeben ist.

5. Katheter (1) nach einem der vorhergehenden Ansprüche, wobei ein Außendurchmesser des Katheterschlauchs (2) höchsten 1 mm beträgt.

6. Nadel-Katheter-Kit zum Verabreichen von Anästhetika an eine periphere Nervenstelle bei einem Patienten, wobei das Kit Folgendes umfasst:
- einen Katheter (1) nach einem der vorhergehenden Ansprüche und mit einem Außendurchmesser, der ein Vorschieben durch die Nadel (11) mit dem Ballon (6) im zusammengefalteten Zustand erlaubt, und
- eine Nadel (11), die dazu angepasst ist, teilweise in einen Patienten eingeführt zu werden und so positioniert zu werden, dass sie von der Außenseite des Patienten und zu der peripheren Nervenstelle, an der die Anästhetika zu verabreichen sind, verläuft, wobei die Nadel (11) einen hohlen Schaft aufweist, durch den der Katheter (1) vorgeschoben werden kann.

7. Nadel-Katheter-Kit zum Verabreichen von Anästhetika an eine periphere Nervenstelle bei einem Patienten, wobei das Kit Folgendes umfasst:
- einen Katheter (1) nach einem der Ansprüche 1-5 und mit einem Innendurchmesser des Katheterschlauchs (2), der das Einführen der Nadel (11) in den Katheterschlauch (2) und das Entfernen der Nadel (11) durch den Katheterschlauch (2) nach dem Positionieren des Katheters (1) in dem Patienten erlaubt, und
- eine Nadel (11), die dazu angepasst ist, teilweise in einen Patienten eingeführt zu werden und so positioniert zu werden, dass sie von der Außenseite des Patienten und zu der peripheren Nervenstelle, an der die Anästhetika zu verabreichen sind, verläuft, wobei die Nadel (11) einen hohlen Schaft aufweist, über den der Katheter (1) angeordnet werden kann.

8. Nadel-Katheter-Kit zum Verabreichen von Anästhetika an eine periphere Nervenstelle bei einem Patienten, wobei das Kit Folgendes umfasst:
- eine Einführungsvorrichtung (20), umfassend einen hohlen schlauchförmigen Einführungsvorrichtungsschlauch (21), wobei der Einführungsvorrichtungsschlauch (21) einen Innendurchmesser aufweist, der das Einführen der Nadel (11) in den Einführungsvorrichtungsschlauch (21) und das Entfernen der Nadel (11) durch den Einführungsvorrichtungsschlauch (21) nach dem Positionieren von zumindest einem Teil des Einführungsvorrichtungsschlauchs (21) und einem Teil der Nadel (11) in dem Patienten ermöglicht,
- einen Katheter (1) nach einem der Ansprüche 1-5 und mit einem Außendurchmesser, der ein Vorschieben durch den Einführungsvorrichtungsschlauch (21) mit dem Ballon (6) im zusammengefalteten Zustand erlaubt, und
- eine Nadel (11), die dazu angepasst ist, in den Einführungsvorrichtungsschlauch (21) eingeführt zu werden und teilweise in den Patienten eingeführt zu werden und so positioniert zu werden, dass sie von der Außenseite des Patienten zu der peripheren Nervenstelle, an der die Anästhetika zu verabreichen sind, verläuft.

## Revendications

1. Cathéter (1) destiné à administrer des anesthésiques à un site nerveux périphérique dans un patient, le cathéter (1) comprenant :
- un tube de cathéter souple et creux (2) possédant une première lumière interne (3) s'étendant le long d'un axe longitudinal du tube de cathéter (2) à partir d'une extrémité proximale (4) jusqu'à une extrémité distale (5), ladite extrémité distale (5) étant agencée au niveau du site nerveux périphérique lors de l'utilisation du cathéter (1), et
- un ballonnet (6) adapté pour être gonflé à partir d'un état replié jusqu'à un état gonflé par un fluide acheminé à travers le tube de cathéter (2) dans une cavité interne (7) du ballonnet (6),
ledit ballonnet (6) étant agencé au niveau de l'extrémité distale (5) du tube de cathéter (2) et ladite cavité interne (7) du ballonnet (6) étant en communication fluidique avec l'extrémité proximale (4) du tube de cathéter (2) par l'intermédiaire de la lumière interne (3),
ledit tube de cathéter (2) comprenant en outre une seconde lumière interne (10) agencée sensiblement parallèle à la première lumière interne (3) et s'étendant à partir de l'extrémité proximale (4) jusqu'à l'extrémité distale (5) du tube de cathéter (2), et
ledit ballonnet (6) étant agencé plus près de l'extrémité proximale (4) du tube de cathéter (2) qu'un point où les anesthésiques doivent quitter le tube de cathéter (2) lors de l'utilisation du cathéter.

2. Cathéter (1) destiné à administrer des anesthésiques à un site nerveux périphérique dans un patient, le cathéter (1) comprenant :
- un tube de cathéter souple et creux (2) possédant une première lumière interne (3) s'étendant le long d'un axe longitudinal du tube de cathéter (2) à partir d'une extrémité proximale (4) jusqu'à une extrémité distale (5), ladite extrémité distale (5) étant agencée au niveau du site nerveux périphérique lors de l'utilisation du cathéter (1), et
- un ballonnet (6) adapté pour être gonflé à partir d'un état replié jusqu'à un état gonflé par un fluide acheminé à travers le tube de cathéter (2) dans une cavité interne (7) du ballonnet (6),
ledit ballonnet (6) étant agencé au niveau de l'extrémité distale (5) du tube de cathéter (2) et ladite cavité interne (7) du ballonnet (6) étant en communication fluidique avec l'extrémité proximale (4) du tube de cathéter (2) par l'intermédiaire de la lumière interne (3),
ledit tube de cathéter (2) comprenant en outre des perforations (8) dans une paroi le long d'une section proche de l'extrémité distale (5) et entourées par le ballonnet (6) de sorte que les anesthésiques puissent s'écouler à partir du tube de cathéter (2) et dans le ballon (6) à travers les perforations (9), et
ledit tube de cathéter (2) comprenant en outre au moins un trou d'extrémité (9) au niveau de l'extrémité distale (5), le trou d'extrémité (9) étant sensiblement plus petit que les perforations (8) dans la paroi et n'étant pas entouré par le ballonnet (6) afin que l'injection d'anesthésiques entraîne le gonflage du ballon (6) jusqu'à ce qu'une certaine pression soit atteinte, après quoi des anesthésiques ensuite administrés quitte la lumière interne (3) à travers le trou d'extrémité (9).

3. Cathéter (1) selon la revendication 1 ou 2, ladite première lumière interne (3) étant la seule lumière s'étendant le long de l'axe longitudinal du tube de cathéter (2).

4. Cathéter (1) selon la revendication 1, ledit tube de cathéter (2) étant imperméable aux anesthésiques le long de toute la longueur afin que les anesthésiques ne puissent quitter le tube de cathéter (2) qu'à travers un trou d'extrémité (9) au niveau de l'extrémité distale (5), le trou d'extrémité (9) n'étant pas entouré par le ballonnet (6).

5. Cathéter (1) selon l'une quelconque des revendications précédentes, un diamètre externe du tube de cathéter (2) étant inférieur ou égal à 1 mm.

6. Ensemble aiguille-cathéter destiné à administrer des anesthésiques à un site nerveux périphérique dans un patient, l'ensemble comprenant :
- un cathéter (1) selon l'une quelconque des revendications précédentes et possédant un diamètre externe qui permet une avance à travers l'aiguille (11) avec le ballonnet (6) à l'état replié, et
- une aiguille (11) adaptée pour être partiellement insérée dans un patient et positionnée afin qu'elle s'étende à partir de l'extérieur du patient et jusqu'au site nerveux périphérique où les anesthésiques doivent être administrés, l'aiguille (11) possédant un corps creux à travers lequel le cathéter (1) peut être avancé.

7. Ensemble aiguille-cathéter destiné à administrer des anesthésiques à un site nerveux périphérique dans un patient, l'ensemble comprenant :
- un cathéter (1) selon l'une quelconque des revendications 1 à 5 et possédant un diamètre interne du tube de cathéter (2) qui permet l'insertion de l'aiguille (11) dans le tube de cathéter (2) et l'enlèvement de l'aiguille (11) à travers le tube de cathéter (2) après le positionnement du cathéter (1) dans le patient, et
- une aiguille (11) adaptée pour être partiellement insérée dans un patient et positionnée afin qu'elle s'étende à partir de l'extérieur du patient et jusqu'au site nerveux périphérique où les anesthésiques doivent être administrés, l'aiguille (11) possédant un corps creux sur lequel le cathéter (1) peut être agencé.

8. Ensemble aiguille-cathéter destiné à administrer des anesthésiques à un site nerveux périphérique dans un patient, l'ensemble comprenant :
- un introducteur (20) comprenant un tube introducteur tubulaire creux (21), le tube introducteur (21) possédant un diamètre interne permettant l'insertion de l'aiguille (11) dans le tube introducteur (21) et le retrait de l'aiguille (11) à travers le tube introducteur (21) après le positionnement d'au moins une partie du tube introducteur (21) et une partie de l'aiguille (11) dans le patient,
- un cathéter (1) selon l'une quelconque des revendications 1 à 5 et possédant un diamètre externe qui permet une avance à travers le tube introducteur (21) avec le ballonnet (6) à l'état replié, et
- une aiguille (11) adaptée pour être insérée dans le tube introducteur (21) et partiellement insérée dans le patient et positionnée afin qu'elle s'étende à partir de l'extérieur du patient et jusqu'au site nerveux périphérique où les anesthésiques doivent être administrés.
